# EUROPEAN PATENT APPLICATION

(11) **EP 2 990 099 A1**
(43) Date of publication of application: **02.03.2016**
(21) Application number: 15180246.9
(22) Date of filing: 07.08.2015
(51) Int. Cl.: B01D 63/16, A61M 1/36, B01D 69/02

(54) **ANTI-THROMBOGENIC POROUS MEMBRANES AND METHODS FOR MANUFACTURING SUCH MEMBRANES**

(30) Priority: 18.08.2014 US 201462038409 P
(71) Applicant: Fenwal, Inc., Lake Zurich, IL 60047 (US)
(72) Inventor: Mizobouchi, Yoshikazu, Mundelein, IL Illinois 60060 (US)
(74) Representative: Jenkinson, Kay Elizabeth

(57) **Abstract**

A system is provided for separating a bodily fluid, such as blood, into two or more constituent parts. The system is configured to cooperate with a fluid flow circuit including a spinning membrane-type fluid separation chamber having a housing, with a fluid inlet port and at least one fluid outlet port. A rotor is positioned within the housing so as to define a gap between the rotor and an inside surface of the housing, with the rotor being rotatable relative to the housing about an axis. A membrane is mounted on the rotor and/or on the inside surface of the housing and faces the gap to separate bodily fluid within the housing into two or more constituent parts. The membrane includes an anti-thrombogenic material, which may be either incorporated into the material of the membrane or coated onto at least a portion of the membrane.

## Description

### Background

### Field of the Disclosure

The invention relates to fluid separation systems and methods. More particularly, the invention relates to systems employing spinning membranes for fluid separation and methods for manufacturing such membranes.

### Description of Related Art

Various blood processing systems now make it possible to collect particular blood constituents, instead of whole blood, from a blood source such as, but not limited to, a container of previously collected blood or other living or non-living source. Typically, in such systems, whole blood is drawn from a blood source, a particular blood component or constituent is separated, removed, and collected, and the remaining blood constituents are returned to the blood source. Removing only particular constituents is advantageous when the blood source is a human donor, because potentially less time is needed for the donor's body to return to pre-donation levels, and donations can be made at more frequent intervals than when whole blood is collected. This increases the overall supply of blood constituents, such as plasma and platelets, made available for transfer and/or therapeutic treatment.

Whole blood is typically separated into its constituents (e.g., red cells, platelets, and plasma) through centrifugation, such as in the AMICUS® separator from Fenwal, Inc. of Lake Zurich, Illinois, or other centrifugal separation devices, or a spinning membrane-type separator, such as the AUTOPHERESIS-C® and AURORA® devices from Fenwal, Inc.

When blood contacts a foreign substance, a number of adverse reactions (including blood coagulation, protein adhesion, protein activation, platelet activation, etc.) are possible at the interface between the blood and the foreign substance, even when an anticoagulant material is mixed with the blood prior to bringing the blood into the vicinity of the foreign substance. In the case of blood separation systems, surfaces of the various component parts forming the blood flow path may prompt such a reaction, and in systems employing a spinning membrane to separate blood, there is also a risk of such a reaction when the blood is brought into contact with the membrane. Accordingly, the need remains for a spinning membrane-type separation system with a membrane that reduces the risk of such reactions when blood is brought into contact with the membrane.

### Summary

There are several aspects of the present subject matter which may be embodied separately or together in the devices and systems described and claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto.

In one aspect, a fluid separation chamber is provided for a fluid flow circuit that is suitable for use in combination with a fluid separation system to separate a fluid into two or more constituent parts. The fluid separation chamber includes a housing with a fluid inlet port and at least one fluid outlet port. A rotor is positioned within the housing so as to define a gap between the rotor and an inside surface of the housing, with the rotor being rotatable relative to the housing about an axis. A membrane is mounted on the rotor and/or on the inside surface of the housing and faces the gap. The membrane includes an anti-thrombogenic material.

In another aspect, a method is provided for manufacturing a membrane of a spinning membrane-type fluid separation chamber. The method includes adding an anti-thrombogenic material to a polymeric material to form a membrane material. The membrane material is formed into a film, with a plurality of pores also being defined in the film.

In yet another aspect, a method is provided for manufacturing a membrane of a spinning membrane-type fluid separation chamber. The method includes providing a membrane and applying an anti-thrombogenic material to at least a portion of the membrane.

### Brief Description of the Drawings

Fig. 1 is a front perspective view of an exemplary fluid separation system according to an aspect of the present disclosure;
Fig. 2 is a rear perspective view of the fluid separation system of Fig. 1, with a rear door thereof in an open position;
Fig. 3 is a front perspective view of the fluid separation system of Fig. 1, with a fluid flow circuit associated therewith;
Fig. 4 is a front perspective view of a fluid separation chamber of the fluid flow circuit of Fig. 3, with a portion thereof broken away for illustrative purposes;
Fig. 5 is a schematic view of the fluid flow circuit and fluid separation system of Fig. 3, in a fluid draw mode; and
Fig. 6 is a schematic view of the fluid flow circuit and fluid separation system of Fig. 3, in a fluid return mode.

### Description of the Illustrated Embodiments

The embodiments disclosed herein are for the purpose of providing an exemplary description of the present subject matter. They are, however, only exemplary, and the present subject matter may be embodied in various forms. Therefore, specific details disclosed herein are not to be interpreted as limiting the subject matter as defined in the accompanying claims.

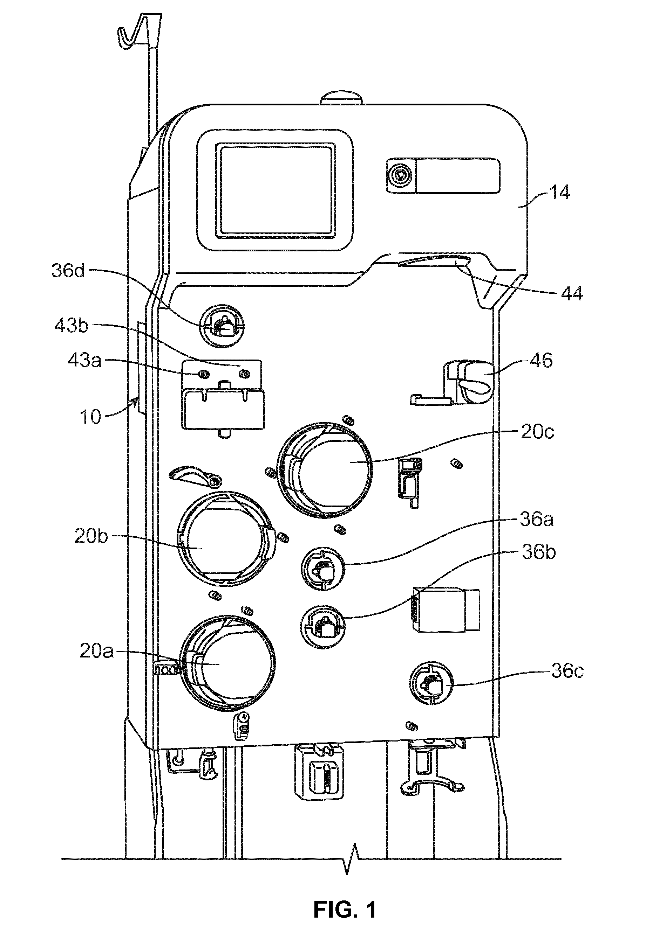

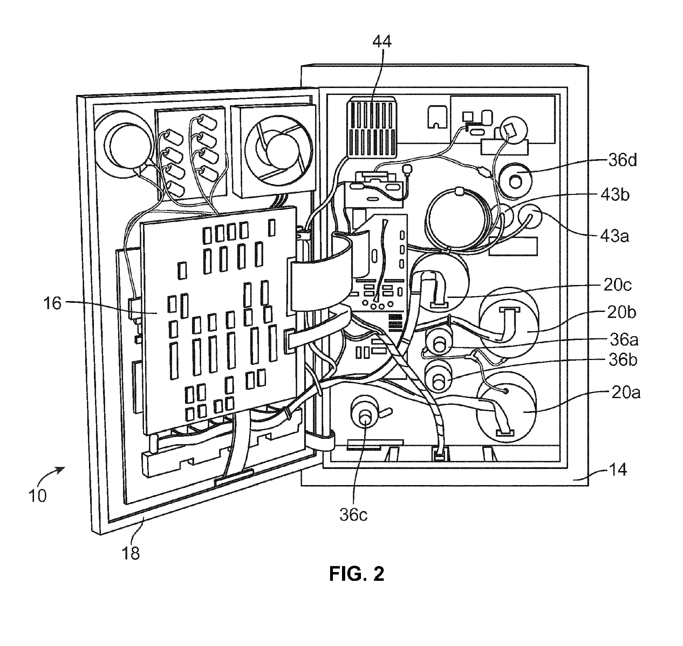

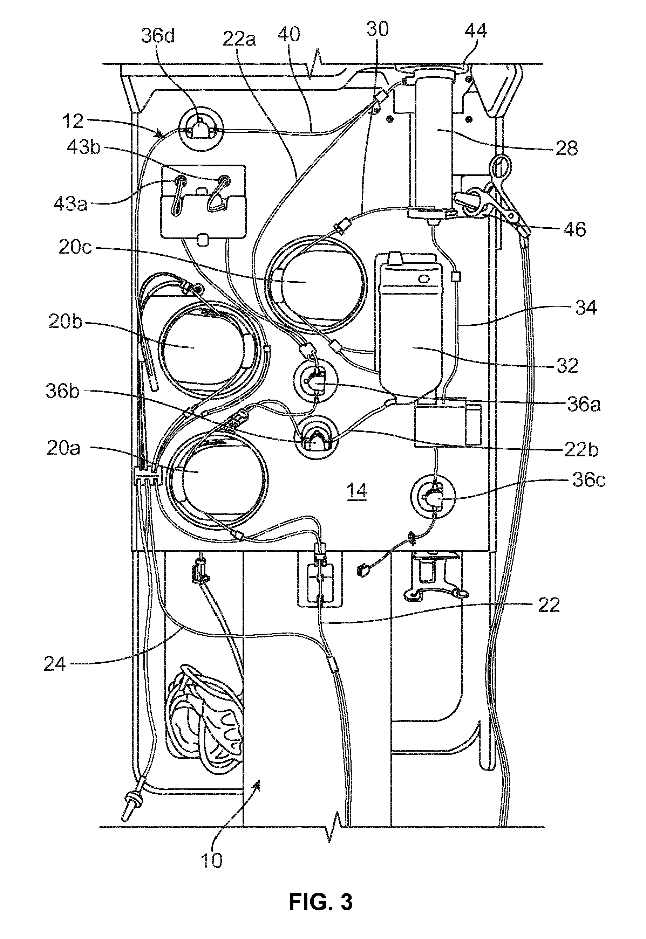

According to an aspect of the present disclosure, a durable or reusable fluid separation system is used in combination with a separate fluid flow circuit (which may be disposable) to separate a fluid into two or more constituent parts. Figs. 1 and 2 illustrate an exemplary fluid separation system 10, while Fig. 3 illustrates an exemplary fluid flow circuit 12 mounted onto the fluid separation system 10, but it should be understood that the illustrated fluid separation system 10 and fluid flow circuit 12 are merely exemplary of such systems and circuits and that differently configured fluid separation systems and fluid flow circuits may be provided without departing from the scope of the present disclosure.

The system 10 of Fig. 1 is configured for processing whole blood, but it may be used to process any other bodily fluid that may have a reaction such as an adverse reaction when brought into contact with a foreign substance (e.g., platelet-rich plasma or red cell concentrate). The fluid may come from any fluid source and be returned to any recipient, which may be the same as or different from the fluid source. In one embodiment, the fluid source/recipient is a living donor or patient (e.g., a human blood donor), while in other embodiments the fluid source and/or fluid recipient may be a non-living source/recipient (e.g., a blood bag or fluid container).

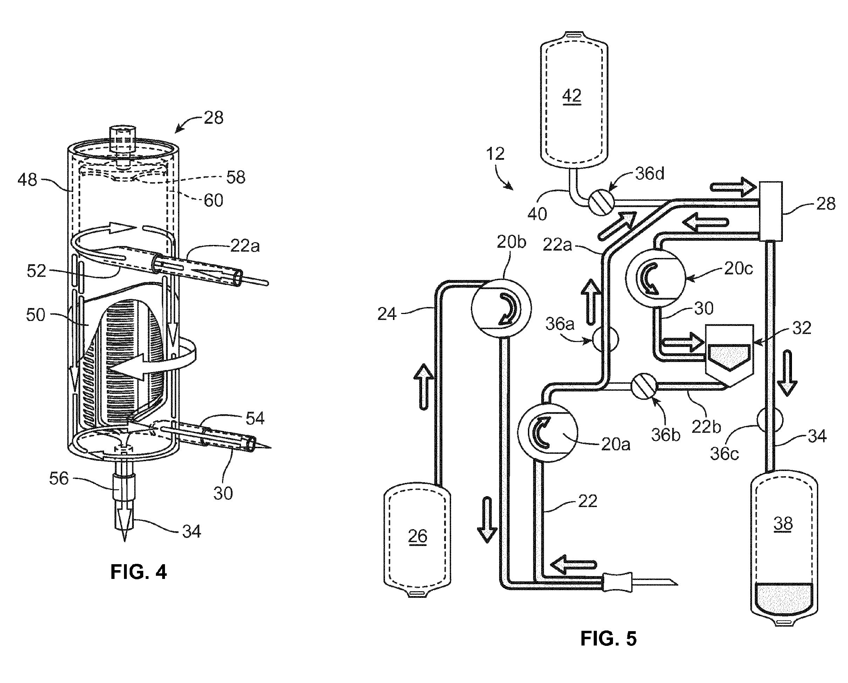

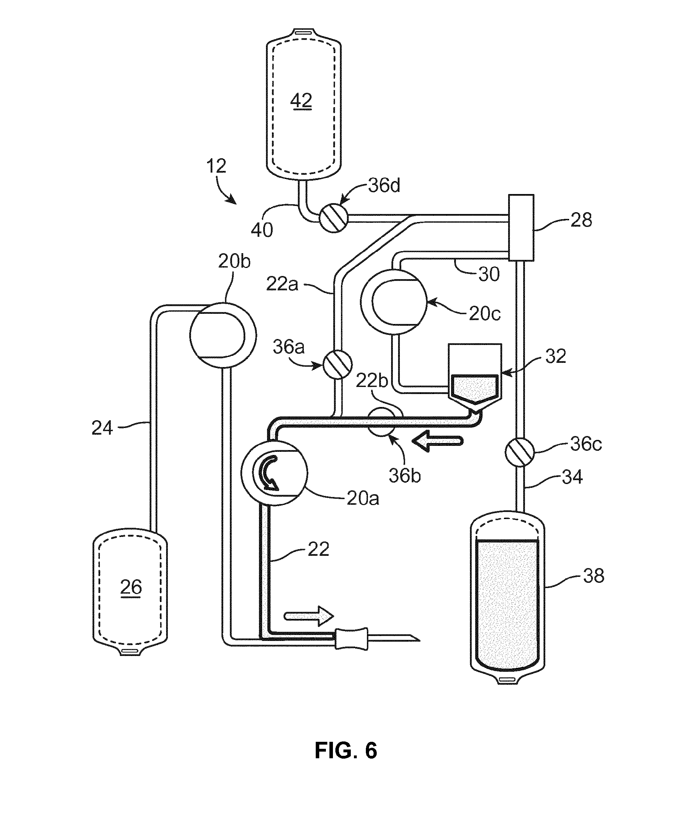

The illustrated system 10 includes a cabinet or housing 14, with several components positioned outside of the cabinet 14 (e.g., associated with a front wall or surface or panel of the cabinet 14) and additional components (including a central processing unit or controller 16) and interconnects positioned inside of the cabinet 14, which may be accessed by opening a rear door 18 of the system 10, as shown in Fig. 2. Among the system components positioned on the outside of the cabinet 14, one or more pumps or pump stations 20a-20c may be provided, with the pumps 20a-20c configured to accommodate tubing lines of the fluid flow circuit 12. One of the pumps 20a may be provided as a source/recipient access pump, which may be associated with a source/recipient access line 22 of the fluid flow circuit 12 and operates to draw fluid from a fluid source (Fig. 5) and to return fluid to a fluid recipient (Fig. 6). Another one of the pumps 20b may be provided as an anticoagulant pump, which may be associated with an anticoagulant line 24 of the fluid flow circuit 12 and operates to add anticoagulant from an anticoagulant source or container 26 of the fluid flow circuit 12 (Fig. 5) to fluid drawn from the fluid source in the source/recipient access line 22 before the fluid enters into a fluid separation module or chamber 28 of the fluid flow circuit 12. A third pump 20c may be provided as a return fluid pump, which may be associated with a return fluid outlet line 30 and operates to draw a return fluid (i.e., a fluid constituent to be returned to a fluid recipient) from the fluid separation chamber 28 and direct it into a return fluid reservoir 32 after the fluid has been separated into a return fluid and a collection fluid in the fluid separation chamber 28.

In the illustrated embodiment, the pumps 20a-20c are peristaltic pumps, but it is within the scope of the present disclosure for differently configured pumps, such as diaphragm or other pumps, to be provided. Furthermore, additional or alternative pumps may be provided without departing from the scope of the present disclosure. For example, a pump may be associated with a collection fluid outlet line 34 of the fluid flow circuit 12 to draw a collection fluid from the fluid separation chamber 28 after the fluid from the fluid source has been separated into a return fluid and a collection fluid. Also, as will be described in greater detail herein, the illustrated embodiment employs a single fluid flow tubing or flow path for both drawing fluid from a source and flowing or returning it to a recipient, which are carried out intermittently. The system 10 could employ separate draw and return flow paths or tubes without departing from the scope of the present disclosure.

In addition to the pumps 20a-20c, the external components of the system 10 may include one or more clamps or valves 36a-36d associated with the tubing lines of the fluid flow circuit 12. The clamps or valves 36a-36d may be variously configured and operate to selectively allow and prevent fluid flow through the associated tubing line. In the illustrated embodiment, one clamp or valve 36a may be provided as a fluid source/recipient clamp, which may be associated with a draw branch 22a of the source/recipient access line 22 of the fluid flow circuit 12 to allow (Fig. 5) or prevent (Fig. 6) the flow of fluid through the draw branch 22a of the source/recipient access line 22. Another one of the clamps or valves 36b may be provided as a reinfusion clamp or valve, which may be associated with a reinfusion branch 22b of the source/recipient access line 22 downstream of a return fluid reservoir 32 of the fluid flow circuit 12 to allow (Fig. 6) or prevent (Fig. 5) the flow of return fluid through the reinfusion branch 22b. A third clamp or valve 36c may be provided as a collection fluid clamp or valve, which may be associated with the collection fluid outlet line 34 to allow (Fig. 5) or prevent (Fig. 6) the flow of collection fluid through the collection fluid outlet line 34 and into a collection fluid container 38. A fourth clamp or valve 36d may be provided as a replacement fluid clamp or valve, which may be associated with a replacement fluid line 40 of the fluid flow circuit 12 to allow or prevent the flow of a replacement fluid out of a replacement fluid source 42 (e.g., a bag or container at least partially filled with saline). Additional or alternative clamps or valves may also be provided without departing from the scope of the present disclosure.

The illustrated system 10 further includes one or more pressure sensors 43a and 43b that may be associated with the fluid flow circuit 12 to monitor the pressure within one or more of the tubing lines of the fluid flow circuit 12 during operation of the pumps 20a-20c and clamps or valves 36a-36d. In one embodiment, one pressure sensor 43a may be associated with a tubing line that draws fluid from a fluid source and/or directs processed fluid to a fluid recipient, while the other pressure sensor 43b may be associated with a tubing line that directs fluid into or out of the fluid separation chamber 28 to assess the pressure within the fluid separation chamber 28, but the pressure sensors 43a and 43b may also be associated with other tubing lines without departing from the scope of the present disclosure. The pressure sensors 43a and 43b may send signals to the system controller 16 that are indicative of the pressure within the tubing line or lines being monitored by the pressure sensor 43a, 43b. If the controller 16 determines that an improper pressure is present within the fluid flow circuit 12 (e.g., a high pressure due to an occlusion of one of the tubing lines), then the controller 16 may instruct one or more of the pumps 20a-20c and/or one or more of the clamps or valves 36a-36d to act so as to alleviate the improper pressure condition (e.g., by reversing the direction of operation of one of the pumps 20a-20c and/or opening or closing one of the clamps or valves 36a-36d). Additional or alternative pressure sensors may also be provided without departing from the scope of the present disclosure.

The system 10 may also include a separation actuator 44 that interacts with a portion of the fluid separation chamber 28 to operate the fluid separation chamber 28. A chamber lock 46 may also be provided to hold the fluid separation chamber 28 in place with respect to the system cabinet 14 and in engagement with the separation actuator 44. The configuration and operation of the separation actuator 44 depends upon the configuration of the fluid separation chamber 28. In the illustrated embodiment, the fluid separation chamber 28 is provided as a spinning membrane-type separator, such as a separator of the type described in greater detail in U.S. Patents Nos. 5,194,145 and 5,234,608 or in PCT Patent Application Publication No. WO 2012/125457 A1, all of which are hereby incorporated herein by reference. If provided as a spinning membrane-type separator, the fluid separation chamber 28 may include a tubular housing 48 (Fig. 4), with a microporous membrane 50 positioned therein. An inlet 52 allows a fluid from a fluid source to enter into the housing 48 (via the draw branch 22a of the source/recipient access line 22), while a side outlet 54 allows return fluid to exit the housing 48 (via the return fluid outlet line 30) and a bottom outlet 56 allows collection fluid to exit the housing 48 (via the collection fluid outlet line 34) after the fluid from the fluid source has been separated into return fluid and collection fluid.

In the illustrated embodiment, the separation actuator 44 is provided as a driver that is magnetically coupled to a rotor 58 on which the membrane 50 is mounted, with the separation actuator 44 causing the rotor 58 and membrane 50 to rotate about the central axis of the housing 48. The rotating rotor 58 and membrane 50 create Taylor vortices within a gap 60 between the housing 48 and the membrane 50, which tend to transport the return fluid away from the membrane 50 to exit the fluid separation chamber 28 via the side outlet 54, while the collection fluid passes through the membrane 50 toward the central axis of the housing 48 to exit the fluid separation chamber 28 via the bottom outlet 56. In one embodiment, whole blood from a blood source is separated into cellular blood components (return fluid) and substantially cell-free plasma (collection fluid). It should be understood that the present disclosure is not limited to a particular fluid separation chamber and that the illustrated and described fluid separation chamber 28 is merely exemplary. For example, in other embodiments, a differently configured spinning membrane-type fluid separation chamber may be employed (e.g., one in which the membrane 50 is mounted on an inside surface of the housing 48 or on both the rotor 58 and an inside surface of the housing 48 and facing the gap 60) without departing from the scope of the present disclosure.

The membrane 50 of the fluid separation chamber 28 may be variously configured without departing from the scope of the present disclosure. When the system 10 is to be used to separate blood into two or more constituents, at least a portion of the membrane 50 preferably has anti-thrombogenic characteristics to prevent or at least decrease the incidence of reaction, such as protein or platelet activation upon the blood being separated within the fluid separation chamber 28. As used herein, the term "anti-thrombogenic" is intended to refer to a substance or property characterized by an enhanced resistance to the accumulation of blood components than the materials typically employed in the manufacture of membranes of spinning membrane-type fluid separation chambers (e.g., nylon 6-6).

Any suitable membrane material (or combination of materials) and anti-thrombogenic material (or combination of materials) may be used in manufacturing the membrane 50. In one embodiment, the membrane 50 is formed of a polymeric material (e.g., nylon 6-6, polyethersulfone, polysulfone, polycarbonate, polyvinylidene fluoride, polyamide, or the like), with an anti-thrombogenic material (e.g., organic substances that contain polyethylene glycol moiety or moieties in their molecular structure, organic substances that contain polyacrylamide moiety or moieties in their molecular structure or polyfluoro organic compounds, or the like) incorporated or mixed or blended therein. In another embodiment, the membrane 50 is fully formed from a polymeric material (e.g., nylon, polyethersulfone, polysulfone, polycarbonate, polyvinylidene fluoride, polyamide, or the like) and then an anti-thrombogenic material (e.g., organic substances that contain polyethylene glycol moiety or moieties in their molecular structure, organic substances that contain polyacrylamide moiety or moieties in their molecular structure or polyfluoro organic compounds, or the like) is applied to or coated onto at least a portion of the formed membrane 50.

A membrane 50 incorporating anti-thrombogenic material into the membrane material may be formed using a solvent exchange procedure. In a solvent exchange procedure, a polymeric material (e.g., polyethersulfone) is dissolved in a first solvent to produce a polymer solution. It may be advantageous to provide the polymeric material in pellet form to increase the rate at which it dissolves in the first solvent. The type of first solvent used may vary depending on the nature of the polymeric material (e.g., formic acid may be used as a first solvent to dissolve a nylon material), although aprotic polar solvents may be non-exclusively preferred over protic solvents.

In the incorporation process, the anti-thrombogenic material (e.g., polyethylene glycol) is also dissolved by the first solvent. The anti-thrombogenic material may be added to the first solvent before adding the polymeric material to the first solvent, after adding the polymeric material to the first solvent, or at the same time as the polymeric material is added to the first solvent. Dissolving both the polymeric material and the anti-thrombogenic material in the first solvent allows the anti-thrombogenic material to mix with the dissolved polymeric material, resulting in a polymer solution having anti-thrombogenic characteristics.

The polymer solution with anti-thrombogenic material incorporated therein may then be treated with a second solvent that is weaker than the first solvent. The second solvent causes the polymer solution to precipitate into the form of a porous membrane material. The type of second solvent used may vary depending on the nature of the polymer solution, but in one embodiment is provided as water.

The membrane material may then be formed into a sheet or film using any suitable techniques to define the membrane 50 that is to be mounted onto the rotor 58 of the fluid separation chamber 28. The dimensions and configuration of the membrane 50 may vary without departing from the scope of the present disclosure, but in one embodiment a membrane 50 formed by a solvent exchange procedure, such as described above, and used for separation of whole blood into plasma and cellular components may have a thickness in the range of approximately 5 µm to approximately 1000 µm (preferably in the range of approximately 25 µm to approximately 200 µm), with a mean pore size in the range of approximately 0.2 µm to approximately 200 (preferably in the range of approximately 0.5 µm to approximately 10 µm and more preferably in the range of approximately 0.6 µm to approximately 5 µm). The porosity of the membrane 50 may also vary, such as from approximately 1% to approximately 90%, but preferably in the range of approximately 50% to approximately 80% to produce a membrane 50 that passes fluid therethrough at a relatively high rate while being sufficiently strong to withstand the forces applied to it by the spinning rotor 58 and fluid contact during a separation procedure.

In alternative embodiments, other methods of manufacturing a membrane 50 incorporating anti-thrombogenic material into the membrane material may be employed. For example, anti-thrombogenic material (e.g., organic substances that contain polyethylene glycol moiety or moieties in their molecular structure) may be added to a polymeric material (e.g., polyethersulfone) that is to be processed and formed into a non-porous film or sheet. The film or sheet may then be processed to add pores to it according to any suitable method. For example, a film may be subjected to a track-etching procedure, whereby regions of increased solubility are formed by exposing the film to highly energized x-ray electrons generated by a synchrotron. A chemical etching step may then be used to remove the regions of the film contacted by the electrons, thereby forming the pores of the resulting membrane 50. According to another alternative approach, the pores of a film or sheet may be formed by a laser ablation process, whereby an excimer laser irradiates the film through openings in a mask, with the portions of the film impinged upon by the laser being formed into pores via ablation. It should be understood that the foregoing methods for manufacturing a membrane impregnated with anti-thrombogenic material are merely exemplary, and other manufacturing methods may be employed without departing from the scope of the present disclosure.

A membrane 50 formed via track-etching or laser ablation may have dimensions and characteristics that vary without departing from the scope of the present disclosure. For example, a membrane 50 formed by a track-etching or laser ablation procedure for separation of whole blood into plasma and cellular components may have a thickness in the range of approximately 5 µm to approximately 1000 µm (preferably in the range of approximately 25 µm to approximately 200 µm and more preferably in the range of approximately 25 µm to approximately 125 µm), with a mean pore size in the range of approximately 0.2 µm to approximately 200 (preferably in the range of approximately 0.5 µm to approximately 10 µm and more preferably in the range of approximately 0.6 µm to approximately 5 µm). The porosity of the membrane 50 formed by track-etching or laser ablation may also vary, such as from approximately 1% to approximately 90%, but preferably in the range of approximately 1% to approximately 20% for a membrane 50 formed by track-etching and in the range of approximately 10% to approximately 50% for a membrane 50 formed by laser ablation.

A membrane 50 formed via a standard solvent exchange procedure (i.e., one in which there is no anti-thrombogenic material incorporated into the polymer solution) or any other suitable approach (e.g., track-etching or laser ablation) may also be provided with anti-thrombogenic properties by applying a coating of anti-thrombogenic material to at least a portion of the membrane 50 (but more preferably to the entire membrane 50). The coating, which may be defined as adhesion and/or covalent chemical bond formation, may be applied by any suitable technique, including (but not limited to) dipping, spraying, spin-coating, vapor deposition, and the like. If necessary, steps may be taken following the coating stage to remove any anti-thrombogenic material that may be blocking or otherwise present in the pores of the membrane 50 (e.g., a laser-ablation procedure that removes excess anti-thrombogenic material from within the pores of the membrane 50). As track-etching and laser ablation remove a portion of the membrane film to define the pores, it may be preferred to use a coating instead of a film impregnated with anti-thrombogenic material to avoid removal of any anti-thrombogenic material from the membrane film.

According to one method of using the fluid separation system 10 and fluid flow circuit 12, a fluid is drawn from a fluid source into the fluid separation chamber 28 during a draw phase or mode (Fig. 5), where it is separated into return fluid (e.g., cellular blood components) and collection fluid (e.g., substantially cell-free plasma). The collection fluid is retained by the system 10, while the return fluid is returned to the fluid source during a return or reinfusion phase or mode (Fig. 6). In one embodiment, the draw and return phases are repeatedly alternated (drawing from the fluid source, separating the fluid from the fluid source into return fluid and collection fluid, and then pumping the collection fluid to the fluid source or a different recipient) until a target (e.g., a particular amount of collection fluid) is achieved. All of the draw phases and all of the return phases may be identical or may differ from each other. For example, a final draw phase may draw less fluid from the fluid source than the previous draw phases and a final return phase may infuse a combination of return fluid and replacement fluid to the fluid recipient, whereas the previous return phases pump only return fluid to the fluid recipient.

It will be understood that the embodiments and examples described above are illustrative of some of the applications of the principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that claims may be directed to the features hereof, including as combinations of features that are individually disclosed or claimed herein.

## Claims

1. A fluid separation chamber (28) of a fluid flow circuit (12) for use in combination with a fluid separation system (10) for separating a fluid into two or more constituent parts, the fluid separation chamber (28) comprising:
a housing (48) including a fluid inlet port (52) and at least one fluid outlet port (54, 56);
a rotor (58) positioned within the housing (48) so as to define a gap (60) between the rotor (58) and an inside surface of the housing (48), wherein the rotor (58) is configured to be rotated relative to the housing (48) about an axis; and
a membrane (50) mounted on the rotor (58) and/or on the inside surface of the housing (48) and facing the gap (60), wherein the membrane (50) includes an anti-thrombogenic material.

2. The fluid separation chamber (28) of claim 1, wherein the membrane (50) is comprised of a polymeric material and said anti-thrombogenic material.

3. The fluid separation chamber (28) of claim 2, wherein the anti-thrombogenic material is coated onto at least a portion of the polymeric material, preferably onto the entire membrane.

4. The fluid separation chamber (28) of claim 3, wherein the polymeric material is coated with the anti-thrombogenic material using a dipping procedure, or a spraying procedure, or a spin-coating procedure, or a vapor deposition procedure.

5. The fluid separation chamber (28) of claim 2, wherein the anti-thrombogenic material is incorporated into the polymeric material.

6. The fluid separation chamber (28) of any of claims 2-5, wherein the polymeric material comprises polyethersulfone.

7. A method of manufacturing a membrane (50) of a spinning membrane-type fluid separation chamber (28), comprising:
adding an anti-thrombogenic material to a polymeric material to form a membrane material;
forming the membrane material into a film;
defining a plurality of pores in the film.

8. The method of claim 7, wherein
said adding an anti-thrombogenic material to a polymeric material to form a membrane material includes dissolving the polymeric material and the anti-thrombogenic material in a first solvent, and
said defining pores in the film includes precipitating the membrane material using a second solvent to form a porous material prior to said forming the membrane material into a film.

9. The method of claim 7, wherein said defining a plurality of pores in the film includes
irradiating the film with electrons generated by a synchrotron to define regions of increased solubility, and
removing the regions of increased solubility via chemical etching to define pores at said regions of increased solubility.

10. The method of claim 7, wherein said defining a plurality of pores in the film includes
providing a mask defining a plurality of openings, and
directing an excimer laser through the openings of the mask to contact the film at a plurality of regions, thereby forming pores at said plurality of regions of the film via laser ablation.

11. The method of any of claims 8-10, wherein the polymeric material comprises polyethersulfone.

12. A method of manufacturing a membrane of a spinning membrane-type fluid separation chamber, comprising:
providing a membrane; and
applying an anti-thrombogenic material to at least a portion of the membrane.

13. The method of claim 12, wherein said applying an anti-thrombogenic material to at least a portion of the membrane includes applying an anti-thrombogenic material to the entire membrane.

14. The method of any of claims 12-13, wherein said applying an anti-thrombogenic material to at least a portion of the membrane includes applying an anti-thrombogenic material to at least a portion of the membrane using a dipping procedure, or a spraying procedure, or a spin-coating procedure, or a vapor deposition procedure.

15. The method of any of claims 12-14, wherein the membrane is comprised of polyethersulfone.
